(19) Europäisches Patentamt
European Patent Office
Office européen des brevets

(11) EP 1 884 229 B1

(12) **FASCICULE DE BREVET EUROPEEN**

(45) Date de publication et mention de la délivrance du brevet:
**21.11.2018 Bulletin 2018/47**

(51) Int Cl.:
***A61K 8/90*** *(2006.01)* ***A61K 8/31*** *(2006.01)*
***A61K 8/81*** *(2006.01)* ***A61Q 1/04*** *(2006.01)*

(21) Numéro de dépôt: **07301256.9**

(22) Date de dépôt: **20.07.2007**

(54) **Composition cosmétique associant un copolymère, une huile non volatile et une huile brillante**

Kosmetische Zusammensetzung enthaltend ein Copolymer, ein nicht-flüchtiges Öl und ein glänzendes Öl

Cosmetic composition associating a copolymer, a non-volatile oil and a shiny oil

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **27.07.2006 FR 0653154**

(43) Date de publication de la demande:
**06.02.2008 Bulletin 2008/06**

(73) Titulaire: **L'Oréal**
**75008 Paris (FR)**

(72) Inventeurs:
- **Lebre-Lemonnier, Caroline**
**75005 Paris (FR)**
- **Boulogne, Sylvie**
**94240, L'Hay Les Roses (FR)**

(74) Mandataire: **Nony**
**11 rue Saint-Georges**
**75009 Paris (FR)**

(56) Documents cités:
**EP-A- 1 411 069** **EP-A- 1 604 634**
**WO-A-02/34218** **FR-A- 2 880 268**
**US-A1- 2005 095 213**

- **DATABASE EPODOC EUROPEAN PATENT OFFICE, THE HAGUE, NL; Jp2006151867 15 juin 2006 (2006-06-15), XP002427666 & JP 2006 151867 A 15 juin 2006 (2006-06-15)**

Remarques:
Le dossier contient des informations techniques présentées postérieurement au dépôt de la demande et ne figurant pas dans le présent fascicule.

EP 1 884 229 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une composition cosmétique de soin et/ou de maquillage de matières kératiniques, en particulier des lèvres.

Les compositions de maquillage et/ou de soin de la peau ou des lèvres contiennent classiquement une phase grasse à base de cire(s) et/ou d'huile(s), des pigments et/ou charges et éventuellement des additifs comme des actifs cosmétiques ou dermatologiques.

Ces compositions sont couramment employées pour procurer une couleur esthétique au support auquel elles sont destinées, voire un effet brillant lorsqu'elles sont plus particulièrement destinées aux lèvres.

Pour procurer cette brillance, les formulateurs de produits cosmétiques mettent généralement en oeuvre des huiles caractérisées par une viscosité et un indice de réfraction élevés et qui possèdent, en outre, de bonnes propriétés de dispersion des pigments ou des charges lorsque ces derniers sont présents dans la composition.

En plus de cet effet coloriel et brillant, l'utilisateur recherche, lors de l'utilisation de ces compositions cosmétiques, des qualités de confort et de longue tenue.

Ainsi, FR 2 880 268 A2 enseigne qu'il est possible d'obtenir des compositions pour les lèvres qui soient brillantes et non transfert grâce à l'incorporation de deux polymères séquencés éthyléniques filmogènes, et EP 1 604 634 A1 qu'une association particulière comprenant au moins un ester de dimère diol avec un diacide dicarboxylique spécifique permet de donner satisfaction en terme de brillance sans par ailleurs affecter la tenue d'une composition de maquillage.

En fait, l'obtention de ces propriétés multiples, à savoir brillance, longue tenue et confort pour un maquillage peut nécessiter la superposition de plusieurs compositions, conditionnées ou non en un unique produit. De tels produits à deux compositions sont notamment décrits dans les documents WO 02/067877 et EP 1 518 534.

Pour des raisons évidentes, cette nécessité de superposer deux compositions peut représenter une contrainte indésirable.

La présente invention a précisément pour but de proposer un nouveau produit cosmétique qui permette d'obtenir en un seul geste un maquillage présentant notamment de bonnes propriétés de confort, une bonne tenue et un effet brillant.

Ainsi, les inventeurs ont découvert qu'il est possible d'obtenir une telle composition sous réserve d'y associer au moins un copolymère spécifique, une huile non volatile et une huile brillante, tels que décrits ci-après.

De façon plus précise selon un premier aspect, la présente invention a pour objet une composition cosmétique comprenant, dans un milieu physiologiquement acceptable, au moins :

- un copolymère séquencé, comprenant au moins une première séquence et au moins une deuxième séquence,
    - la première séquence étant obtenue à partir d'au moins un monomère acrylate de formule CH2 = CH-COOR2 dans laquelle R2 représente un groupe cycloalkyle C4 à C12 et d'au moins un monomère méthacrylate de formule CH2 = C(CH3)-COOR'2 dans laquelle R'2 représente un groupe cycloalkyle C4 à C12,
    - la deuxième séquence étant obtenue à partir d'un monomère acide acrylique et d'au moins un autre monomère de transition vitreuse inférieure ou égale à 20°C choisi parmi,
        - les acrylates de formule CH2 = CHCOOR3, R3 représentant un groupe alkyle non substitué en C1 à C12, linéaire ou ramifié, à l'exception du groupe tertiobutyle, dans lequel se trouve(nt) éventuellement intercalé(s) un ou plusieurs hétéroatomes choisis parmi O, N, S,
        - les méthacrylates de formule CH2 = C(CH3)-COOR4, R4 représentant un groupe alkyle non substitué en C6 à C12 linéaire ou ramifié, dans lequel se trouve(nt) éventuellement intercalé(s) un ou plusieurs hétéroatomes choisis parmi O, N et S;
        - les esters de vinyle de formule R5-CO-O-CH = CH2 où R5 représente un groupe alkyle en C4 à C12 linéaire ou ramifié ;
        - les éthers d'alcool vinylique et d'alcool en C4 à C12,
        - les N-alkyl en C4 à C12 acrylamides, tels que le N-octylacrylamide,
        - et leurs mélanges,
- une huile non volatile ayant une chaine hydrocarbonée comprenant au moins 16 atomes de carbone et ayant une masse molaire inférieure à 650 g/mole, et
- une huile brillante ayant une masse molaire allant de 650 à 10000 g/mole choisie parmi les polybutylènes, les polyisobutylènes hydrogénés, les polydécènes et les polydécènes hydrogénés, les copolymères de la vinylpyrrolidone, les esters d'acide gras

linéaires ayant un nombre total de carbone allant de 35 à 70, les esters hydroxylés, le tridécyl trimellitate, les esters

d'alcool gras ou d'acide gras ramifiés en C4-C28, les huiles siliconées phénylées, l'huile de sésame et leurs mélanges.

L'invention a également pour objet un procédé cosmétique de maquillage des lèvres comprenant l'application sur une matière kératinique et notamment les lèvres d'une composition telle que définie précédemment.

L'invention a également pour objet l'utilisation d'un copolymère séquencé, comprenant au moins une première séquence et au moins une deuxième séquence,

- la première séquence étant obtenue à partir d'au moins un monomère acrylate de formule $CH_2 = CH\text{-}COOR_2$ dans laquelle $R_2$ représente un groupe cycloalkyle $C_4$ à $C_{12}$ et d'au moins un monomère méthacrylate de formule $CH_2 = C(CH_3)\text{-}COOR'_2$ dans laquelle $R'_2$ représente un groupe cycloalkyle $C_4$ à $C_{12}$,
- la deuxième séquence étant obtenue à partir d'un monomère acide acrylique et d'au moins un autre monomère de transition vitreuse inférieure ou égale à 20°C choisi parmi,
  - les acrylates de formule $CH_2 = CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
  - les méthacrylates de formule $CH_2 = C(CH_3)\text{-}COOR_4$, $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi O, N et S;
  - les esters de vinyle de formule $R_5\text{-}CO\text{-}O\text{-}CH = CH_2$ où $R_5$ représente un groupe alkyle en $C_4$ à $C_{12}$ linéaire ou ramifié ;
  - les éthers d'alcool vinylique et d'alcool en $C_4$ à $C_{12}$,
  - les N-alkyl en $C_4$ à $C_{12}$ acrylamides, tels que le N-octylacrylamide,
  - et leurs mélanges,

dans une composition destinée à procurer un dépôt sur les lèvres, doté d'une bonne tenue et d'une brillance satisfaisante.

## COPOLYMERE

Selon l'invention, le copolymère est issu essentiellement de monomères choisis parmi l'acide acrylique, les méthacrylates d'alkyle, les acrylates d'alkyle, et leurs mélanges.

Par « essentiellement », on entend, dans ce qui précède et dans ce qui suit, comprenant au moins 85 %, de préférence au moins 90 %, mieux au moins 95 % et encore mieux 100 %.

Dans ce mode de réalisation, le copolymère a de préférence un poids moléculaire supérieur à 80 000 g/mole.

Le copolymère peut avantageusement comprendre plus de 2 % en poids de monomères acide acrylique, et notamment de 2 à 15 % en poids, par exemple de 3 à 15 % en poids, en particulier de 4 à 15 % en poids, voire de 5 à 10 % en poids de monomères acide acrylique, par rapport au poids total dudit copolymère.

En ce qui concerne les esters acrylates et méthacrylates, ils peuvent dériver de l'estérification d'alcools linéaire ou ramifié, cyclique ou aromatiques en $C_1$ à $C_{12}$, en particulier en $C_4$ à $C_{10}$.

A titre illustratif et non limitatif de ces alcools, on peut notamment citer l'isoborneol.

Selon un mode de réalisation, ledit copolymère comprend au moins des monomères d'acrylates et de méthacrylates dérivant de l'estérification d'un même alcool et en particulier de l'isobornéol.

Selon un autre mode de réalisation, ledit copolymère comprend au moins des monomères de type acrylate d'isobutyle.

Selon l'invention, le copolymère se présente sous la forme d'un copolymère séquencécomprenant au moins une première séquence et au moins une deuxième séquence,
la première séquence étant obtenue à partir d'au moins un monomère acrylate de formule $CH_2 = CH\text{-}COOR_2$ dans laquelle $R_2$ représente un groupe cycloalkyle en C4 à $C_{12}$ et d'au moins un monomère méthacrylate de formule $CH_2 = C(CH_3)\text{-}COOR'_2$ dans laquelle $R'_2$ représente un groupe cycloalkyle $C_4$ à $C_{12}$,
la deuxième séquence étant obtenue à partir d'un monomère acide acrylique et d'au moins un autre monomère de transition vitreuse inférieure ou égale à 20 °C.

Selon un mode de réalisation préféré, $R_2$ et $R'_2$ représentent indépendamment ou simultanément un groupe isobornyle.

Par « au moins » une séquence, on entend une ou plusieurs séquences.

Par copolymère « séquencé », on entend un polymère comprenant au moins 2 séquences distinctes, de préférence au moins 3 séquences distinctes.

La première séquence et la deuxième séquence du copolymère peuvent être avantageusement incompatibles l'une avec l'autre.

Par « séquences incompatibles l'une avec l'autre », on entend que le mélange formé par un polymère correspondant à la première séquence et par un polymère correspondant à la deuxième séquence, n'est pas miscible dans le solvant de polymérisation, majoritaire en poids, du copolymère séquencé, à température ambiante (25 °C) et pression atmosphérique ($10^5$ Pa), pour une teneur du mélange desdits polymères supérieure ou égale à 5 % en poids, par rapport au poids total du mélange desdits polymères et dudit solvant de polymérisation, étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du copolymère séquencé +/- 15 %.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.

Bien entendu, dans le cas d'une polymérisation réalisée dans un solvant unique, ce dernier est le solvant majoritaire.

Lesdites première et deuxième séquences peuvent être avantageusement reliées entre elles par un segment intermédiaire comprenant au moins un monomère constitutif de la première séquence et au moins un monomère constitutif de la deuxième séquence.

Le segment intermédiaire est une séquence comprenant au moins un monomère constitutif de la première séquence et au moins un monomère constitutif de la deuxième séquence du copolymère permet de « compatibiliser » ces séquences.

Avantageusement, le segment intermédiaire comprenant au moins un monomère constitutif de la première séquence et au moins un monomère constitutif de la deuxième séquence du copolymère est un polymère statistique.

De préférence, la séquence intermédiaire est issue essentiellement de monomères constitutifs de la première séquence et de la deuxième séquence.

Avantageusement, la séquence intermédiaire a une température de transition vitreuse Tg comprise entre les températures de transition vitreuse des première et deuxième séquences.

Le copolymère séquencé de la composition selon l'invention est avantageusement un copolymère éthylénique séquencé filmogène.

Par polymère « éthylénique », on entend un polymère obtenu par polymérisation de monomères comprenant une insaturation éthylénique.

Par polymère « filmogène », on entend un polymère apte à former à lui seul ou en présence d'un agent auxiliaire de filmification, un film continu et adhérent sur un support, notamment sur les matières kératiniques.

De façon préférentielle, le copolymère ne comprend pas d'atomes de silicium dans son squelette. Par « squelette », on entend la chaîne principale du copolymère, par opposition aux chaînes latérales pendantes.

De préférence, le copolymère n'est pas hydrosoluble, c'est à dire que le copolymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1 % en poids, à température ambiante (25 °C).

De préférence, le copolymère n'est pas un élastomère.

Par « copolymère non élastomère », on entend un polymère qui, lorsqu'il est soumis à une contrainte visant à l'étirer (par exemple de 30 % relativement à sa longueur initiale), ne revient pas à une longueur sensiblement identique à sa longueur initiale lorsque cesse la contrainte.

De manière plus spécifique, par « copolymère non élastomère » on désigne un polymère ayant une recouvrance instantanée $R_i$ < à 50 % et une recouvrance retardée $R_{2h}$ < 70 % après avoir subi un allongement de 30 %. De préférence, $R_i$ est < à 30 %, et $R_{2h}$ < 50 %.

Plus précisément, le caractère non élastomérique du copolymère est déterminé selon le protocole suivant :
On prépare un film de copolymère par coulage d'une solution du copolymère dans une matrice téflonnée puis séchage pendant 7 jours dans une ambiance contrôlée à 23±5 °C et 50±10 % d'humidité relative.

On obtient alors un film d'environ 100 μm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)

- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i) / \varepsilon_{max}) \times 100$$

Pour déterminer la recouvrance retardée, on mesure après 2 heures le taux d'allongement résiduel de l'éprouvette en pourcentage ($\varepsilon_{2h}$), 2 heures après retour à la contrainte charge nulle.
La recouvrance retardée en % ($R_{2h}$) est donnée par la formule ci-après:

$$R_{2h} = (\varepsilon_{max} - \varepsilon_{2h}) / \varepsilon_{max}) \times 100$$

A titre purement indicatif, le copolymère possède de préférence une recouvrance instantanée $R_i$ de 10 % et une recouvrance retardée $R_{2h}$ de 30 %.
L'indice de polydispersité du copolymère est avantageusement supérieur à 2.
L'indice de polydispersité I du copolymère est égal au rapport de la masse moyenne en poids Mw sur la masse moyenne en nombre Mn.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).
La masse moyenne en poids (Mw) du copolymère est de préférence inférieure ou égale à 300 000 g/mol, elle va par exemple de 35 000 à 200 000 g/mol, et mieux de 45 000 à 150 000 g/mol.
La masse moyenne en nombre (Mn) du copolymère est de préférence inférieure ou égale à 70 000 g/mol, elle va par exemple de 10 000 à 60 000 g/mol, et mieux de 12 000 à 50 000 g/mol.
De préférence, l'indice de polydispersité du copolymère est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.
Le copolymère séquencé comprend au moins une première séquence et au moins une deuxième séquence.
La première séquence est obtenue à partir d'au moins un monomère acrylate de formule $CH_2 = CH\text{-}COOR_2$ et d'au moins un monomère méthacrylate de formule $CH_2 = C(CH_3)\text{-}COOR_2$ dans laquelle $R_2$ représente un groupe cycloalkyle $C_4$ à $C_{12}$. Les monomères et leurs proportions sont de préférence choisis de telle sorte que la température de transition vitreuse de la première séquence est supérieure à 20 °C.
La deuxième séquence est obtenue à partir d'un monomère acide acrylique et d'au moins un monomère de transition vitreuse inférieure ou égale à 20 °C.
Les monomères et leurs proportions sont de préférence choisis de telle sorte que la température de transition vitreuse de la deuxième séquence est inférieure ou égale à 20 °C.
Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox : $1/Tg = \Sigma_\iota (\varpi_i / Tg_i)$, $\varpi_i$ étant la fraction massique du monomère i dans la séquence considerée et $Tg_i$ étant la température de transition vitreuse de l'homopolymère du monomère i.
Sauf indication contraire, les Tg indiquées pour les première et deuxième séquences dans la présente demande sont des Tg théoriques.
L'écart entre les températures de transition vitreuse des première et deuxième séquences est généralement supérieur à 10 °C, de préférence supérieur à 20 °C, et mieux supérieur à 30 °C.
On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

Première séquence

La première séquence a de préférence une Tg supérieure à 20 °C par exemple une Tg allant de 20 à 170 °C,

de préférence supérieure ou égale à 50 °C, allant par exemple de 50 °C à 160 °C, notamment allant de 90 °C à 130 °C.

Selon l'invention, la première séquence est obtenue à partir d'au moins un monomère acrylate de formule $CH_2 = CH\text{-}COOR_2$ dans laquelle $R_2$ représente un groupe cycloalkyle $C_4$ à $C_{12}$, et d'au moins un monomère méthacrylate de formule $CH_2 = C(CH_3)\text{-}COOR'_2$ dans laquelle $R'_2$ représente un groupe cycloalkyle $C_4$ à $C_{12}$.

La première séquence peut être obtenue exclusivement à partir dudit monomère acrylate et dudit monomère méthacrylate.

Le monomère acrylate et le monomère méthacrylate sont de préférence dans des propositions massiques comprises entre 30:70 et 70:30, de préférence entre 40:50 et 50:40, notamment de l'ordre de 50:50.

La proportion de la première séquence va avantageusement de 20 à 90 % en poids par rapport au poids total du copolymère, mieux de 30 à 80 % en poids et encore mieux de 60 à 80 % en poids.

Selon un mode de mise en oeuvre, la première séquence est obtenue par polymérisation du méthacrylate d'isobornyle et de l'acrylate d'isobornyle.

La première séquence peut en outre comprendre :

- de l'acide (méth)acrylique, de préférence de l'acide acrylique,
- de l'acrylate de tertiobutyle
- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle,
  - les (méth)acrylamides de formule :

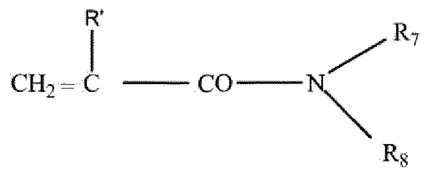

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyle,
et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide,

- et leurs mélanges.

Deuxième séquence

La deuxième séquence a avantageusement une température de transition vitreuse Tg inférieure ou égale à 20 °C, par exemple une Tg allant de -100 à 20 °C, de préférence inférieure ou égale à 15 °C, notamment allant de -80 °C à 15 °C et mieux inférieure ou égale à 10 °C, par exemple allant de -100 °C à 10 °C, notamment allant de -30 °C à 10 °C.

La deuxième séquence est obtenue à partir d'un monomère acide acrylique et d'au moins un autre monomère ayant une Tg inférieure ou égale à 20 °C.

Le monomère de transition vitreuse inférieure ou égale à 20 °C est, de préférence, choisi parmi les monomères suivants:

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S, tel qu'un isobutyle,
- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi O, N et S;
- les esters de vinyle de formule $R_5\text{-}CO\text{-}O\text{-}CH = CH_2$
  où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,

- et leurs mélanges.

Ils peuvent de préférence être choisis parmi des acrylates de formule $CH_2$ = $CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S, et en particulier avec $R_3$ représentant l'isobutyle.
Les monomères ayant une Tg inférieure ou égale à 20 °C préférés sont l'acrylate d'isobutyle, l'acrylate d'éthyl-2 hexyle ou leurs mélanges en toute proportions.
Chacune des première et deuxième séquences peut contenir en proportion minoritaire au moins un monomère constitutif de l'autre séquence.
Ainsi la première séquence peut contenir au moins un monomère constitutif de la deuxième séquence et inversement.
Chacune des première et/ou deuxième séquence, peu(ven)t comprendre, outre les monomères indiqués ci-dessus, un ou plusieurs autres monomères appelés monomères additionnels, différents des monomères principaux cités précédemment.
La nature et la quantité de ce ou ces monomères additionnels sont choisies de manière à ce que la séquence dans laquelle ils se trouvent ait la température de transition vitreuse désirée.
Ce monomère additionnel est par exemple choisi parmi :

- les monomères à insaturation(s) éthylénique(s) comprenant au moins une fonction aminé tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2$ = $C(CH_3)$-$COOR_6$
  dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule $CH_2$ = $C(CH_3)$-$COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi O, N et S, ledit groupe alkyle étant substitué par un ou plusieurs substituants choisis parmi les groupes hydroxyle et les atomes d'halogènes (Cl, Br, I, F) ;
- les acrylates de formule $CH_2$ = $CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène.

Le monomère additionnel peut représenter 0,5 à 30 % en poids du poids du copolymère. Selon un mode de mise en oeuvre, le copolymère ne contient pas de monomère additionnel.
De préférence, le copolymère comprend au moins des monomères acrylate d'isobornyle et méthacrylate d'isobornyle dans la première séquence et des monomères acrylate d'isobutyle et acide acrylique dans la deuxième séquence.
De préférence, le copolymère comprend au moins des monomères acrylate d'isobornyle et méthacrylate d'isobornyle en proportion équivalente en poids dans la première séquence et des monomères acrylate d'isobutyle et acide acrylique dans la deuxième séquence.
De préférence, le copolymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la première séquence représentant 70 % en poids du copolymère.
De préférence, le copolymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la séquence de Tg supérieure à 20 °C représentant 70 % en poids du copolymère, et l'acide acrylique représentant 5 % en poids du copolymère.
Selon un mode préféré de réalisation, le copolymère comprend de 50 à 80 % en poids de méthacrylate/acrylate d'isobornyle, de 10 à 30 % en poids d'acrylate d'isobutyle et de 2 à 10 % en poids d'acide acrylique, par rapport au poids total du copolymère.
Lorsque le copolymère conforme à l'invention comprend au moins un monomère acide acrylique, le copolymère peut être préparé par un procédé consistant à mélanger, dans un même réacteur, un solvant de polymérisation, un amorceur, un monomère acide acrylique, au moins un monomère de transition vitreuse inférieure ou égale à 20 °C, au

moins un monomère acrylate de formule $CH_2$ = CH-$COOR_2$ dans laquelle $R_2$ représente un groupe cycloalkyle $C_4$ à $C_{12}$, et au moins un monomère méthacrylate de formule $CH_2$ = $C(CH_3)$-COOR'$_2$ dans laquelle R'$_2$ représente un groupe cycloalkyle $C_4$ à $C_{12}$, selon la séquence d'étape suivante :

- on verse dans le réacteur, une partie du solvant de polymérisation et une partie de l'amorceur, mélange que l'on chauffe à une température de réaction comprise entre 60 et 120 °C,
- on verse ensuite, en une première coulée, ledit au moins monomère acrylate de formule $CH_2$ = CH-$COOR_2$ et ledit au moins monomère méthacrylate de formule $CH_2$ = $C(CH_3)$-COOR'$_2$ que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, le monomère acide acrylique et ledit au moins monomère de transition vitreuse inférieure ou égale à 20 °C, qu'on laisse réagir pendant une durée T' au bout de laquelle le taux de conversion desdits monomères atteint un plateau,
- on ramène le mélange réactionnel à température ambiante.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

Selon un autre mode de mise en oeuvre, le copolymère peut être préparé selon un procédé de préparation, consistant à mélanger, dans un même réacteur, un solvant de polymérisation, un amorceur, un monomère acide acrylique, au moins un monomère de transition vitreuse inférieure ou égale à 20 °C, au moins un monomère acrylate de formule CH2 = CH-$COOR_2$ dans laquelle $R_2$ représente un groupe cycloalkyle $C_4$ à $C_{12}$ et au moins un monomère méthacrylate de formule $CH_2$ = $C(CH_3)$-COOR'$_2$ dans laquelle R'$_2$ représente un groupe cycloalkyle $C_4$ à $C_{12}$, selon la séquence d'étape suivante :

- on verse dans le réacteur, une partie du solvant de polymérisation et une partie de l'amorceur, mélange que l'on chauffe à une température de réaction comprise entre 60 et 120 °C,
- on verse ensuite, en une première coulée, le monomère acide acrylique et ledit au moins monomère de transition vitreuse inférieure ou égale à 20 °C que l'on laisse à réagir pendant une durée T correspondant à un taux de conversion desdits monomères de 90 % maximum,

- on verse ensuite dans le réacteur, en une deuxième coulée, à nouveau de l'amorceur de polymérisation, ledit au moins monomère acrylate de formule $CH_2$ = CH-$COOR_2$ et ledit au moins monomère méthacrylate de formule $CH_2$ = $C(CH_3)$-COOR'$_2$, qu'on laisse réagir pendant une durée T' au bout de laquelle le taux de conversion desdits monomères atteint un plateau,
- on ramène le mélange réactionnel à température ambiante.

La température de polymérisation est de préférence de l'ordre de 90 °C.

La durée de réaction après la deuxième coulée est de préférence comprise entre 3 et 6 heures.

Les monomères mis en oeuvre dans le cadre de ce procédé, ainsi que leurs proportions peuvent être ceux et celles décrites précédemment dans le paragraphe relatif au copolymère.

Le copolymère entrant dans la composition selon l'invention peut être le copolymère susceptible d'être obtenu par le procédé décrit précédemment.

La composition selon l'invention comprend moins de 40 % en poids de matière active de copolymère, et avantageusement de 5 à 40 % en poids, notamment de 5 à 30 % en poids voire de 10 à 20 % en poids, par rapport au poids total de la composition.

## HUILE NON VOLATILE

La composition selon l'invention comprend avantageusement une huile non volatile hydrocarbonée comprenant une chaîne d'au moins 16 atomes de carbone et ayant une masse molaire inférieure à 650 g/mole.

Par hydrocarbonée, on entend une chaîne constituée essentiellement de carbone et d'hydrogène et pouvant contenir au moins un hétéroatome tel que un oxygène, un halogène ou un azote.

L'huile hydrocarbonée est de préférence constituée de carbone et d'hydrogène.

Par « huile », on entend un composé non aqueux, liquide à température ambiante (25 °C) et pression atmosphérique (760 mm de Hg).

L'huile non volatile selon l'invention peut notamment comprendre une huile ayant une viscosité comprise entre 10 et 300 cPs, de préférence entre 15 et 200 cPs et restant sur la peau ou la fibre kératinique, plus généralement sur

la matière kératinique, à température ambiante et pression atmosphérique, au moins plusieurs heures et ayant notamment une pression de vapeur inférieure à $10^{-3}$ mm de Hg (0,13 Pa). Cette sous-famille d'huile est particulièrement avantageuse en terme de confort pour l'utilisateur notamment au regard de sa fluidité. Ces huiles se prêtent en effet à un étalement aisé et contrôlé.

La viscosité de l'huile non volatile est mesurée à 25 °C à l'aide du Rhéomètre HAAKE RS75 équipé du mobile cône-plan dit 60/2°.

Comme la viscosité de l'huile est relativement faible, on choisit un plan de 60 mm et un mobile de même diamètre dont la troncature est de 200 microns, ce qui correspond à un angle de 2° (d'où la référence 60/2° du mobile).

On dépose une quantité d'huile en excès sur le plan de manière à ce que l'entrefer (espace entre le mobile et le plan d'une épaisseur de 200 microns et d'un diamètre de 60 mm) soit occupé entièrement par ladite huile.

On procède ensuite à un balayage en contrainte (par exemple de $10^{-2}$ à 1000 Pa). L'huile selon l'invention étant un liquide newtonien, on obtient une valeur constante de la viscosité quelque soit la contrainte exercée.

L'huile peut plus particulièrement comprendre une huile hydrocarbonée, linéaire ou ramifiée ayant une masse molaire comprise entre 100 et 650 g/mole et plus particulièrement entre 200 et 650 g/mole.

A titre illustratif et non limitatif des ces huiles, on peut plus particulièrement citer les polyalcènes et en particulier les polybutylènes tel que l'Indopol H15®, les polydécènes tel que le Silkflo 366®, les alcanes et en particulier le squalane tel que la Phytosqualane®, le Sophiderm® ou le Fitoderm® ou les isoparaffines hydrogénées tel que le Parléam®, l'isoeicosane et leurs mélanges.

L'huile non volatile peut être en particulier le squalane.

Selon un mode de réalisation particulier, la composition selon l'invention comprend une association de squalane et d'un copolymère méthacrylate d'isobornyle/acrylate d'isobornyle/acrylate d'isobutyle/acide acrylique.

La composition selon l'invention peut comprendre de 2 à 50 % en poids d'huile non volatile, notamment de 5 à 20 %, par rapport au poids total de la composition.

## HUILE BRILLANTE

La composition selon l'invention comprend avantageusement en outre une huile brillante différente de l'huile non volatile.

Par « huile brillante » on entend une huile apte à procurer un effet de brillance au niveau de la composition l'incorporant.

D'une manière générale, les choix de l'huile brillante et de sa quantité dans la composition selon l'invention peuvent être effectués de manière à procurer à la composition une brillance moyenne mesurée à 20° supérieure ou égale à 35, par exemple 40, de préférence 45, 55, 60 ou 65 sur 100, et/ou une brillance moyenne mesurée à 60° supérieure ou égale à 65, 70, 75 ou 80 sur 100.

Par « brillance moyenne », on désigne la brillance telle qu'elle peut être mesurée à l'aide d'un brillancemètre, de manière conventionnelle comme indiqué ci-dessous.

On procède à la mesure de la brillance moyenne à 20 ° comme suit.

Sur une carte de contraste de marque LENETA et de référence FORM 1A PENOPAC, on étale une couche comprise entre 50 $\mu$m et 150 $\mu$m d'épaisseur de la composition à l'aide d'un étaleur automatique. La couche recouvre au moins le fond blanc de la carte. On laisse sécher le dépôt 24 heures à une température de 30 °C, puis on procède à la mesure de la brillance à 20° sur le fond blanc à l'aide d'un brillancemètre de marque BYK GARDNER et de référence microTRI-GLOSS.

Cette mesure (comprise entre 0 et 100) est répétée au moins trois fois, et la brillance moyenne est la moyenne des au moins trois mesures effectuées.

La brillance moyenne à 60 ° est mesurée de manière similaire en effectuant la mesure à 60 ° plutôt qu'à 20 °.

Les huiles susceptibles d'être mises en oeuvre pour procurer cet effet de brillance peuvent avoir une masse molaire allant en particulier de 650 à 10 000 g/mole, et de préférence de 750 à 7 500 g/mole.

L'huile de masse molaire allant de 650 à 10000 g/mole peut être choisie parmi :

- les polymères lipophiles tels que :
  - les polybutylènes tels que L'INDOPOL H-100 (de masse molaire ou MM=965 g/mole), L'INDOPOL H-300 (MM=1340 g/mole), L'INDOPOL H-1500 (MM=2160g/mole) commercialisés ou fabriqués par la société AMOCO,
  - les polyisobutylènes hydrogénés tels que le PANALANE H-300 E commercialisés ou fabriqué par la société AMOCO (MM =1340 g/mole), le VISEAL 20000 commercialisé ou fabriqué par la société SYNTEAL (MM=6000 g/mole), le REWOPAL PIB 1000 commercialisé ou fabriqué par la société WITCO (MM=1000 g/mole),
  - les polydécènes et les polydécènes hydrogénés tels que : le PURESYN 10 (MM=723 g/mole), le PURESYN 150 (MM=9200 g/mole) commercialisés ou fabriqués par la société MOBIL CHEMICALS,

- les copolymères de la vinylpyrrolidone tels que : le copolymère vinylpyrrolidone/1-héxadécène, ANTARON V-216 commercialisé ou fabriqué par la société ISP (MM=7300 g/mole),

- les esters tels que :

  - les esters d'acides gras linéaires ayant un nombre total de carbone allant de 35 à 70 comme le tétrapélargonate de pentaérythrityle (MM=697,05 g/mole),
  - les esters hydroxylés tels que le triisostéarate de polyglycérol-2 (MM=965,58 g/mole),
  - les esters aromatiques tels que le tridécyl trimellitate (MM=757,19 g/mole),
  - les esters d'alcool gras ou d'acides gras ramifiés en $C_{24}$-$C_{28}$ tels que ceux décrits dans la demande EP-A-0 955 039, et notamment le citrate de triisoarachidyle (MM=1033,76 g/mole), le tétraisononanoate de pentaérythrityle (MM=697,05g/mole), le triisostéarate de glycéryle (MM=891,51 g/mole), le tri décyl-2 tétradécanoate de glycéryle (MM=1143,98 g/mole), le tétraisostéarate de pentaérythrityle (MM=1202,02 g/mole), le tétraisostéarate de polyglycéryle-2 (MM=1232,04 g/mole) ou encore le tétra décyl -2 tétradécanoate de pentaérythrityle (MM=1538,66 g/mole),

- les huiles siliconées telles que les silicones phénylées comme la BELSIL PDM 1000 de la société WACKER (MM=9000 g/mole),
- les huiles d'origine végétale telles que l'huile de sésame (820,6 g/mole),
- et leurs mélanges.

Selon un mode de réalisation particulier, la composition comprend à titre d'huile brillante, au moins un polymère lipophile et en particulier du polybutylène.

Selon un mode de réalisation particulier, la composition selon l'invention comprend une association de polybutylène et d'un copolymère méthacrylate d'isobornyle/acrylate d'isobornyle/acrylate d'isobutyle/acide acrylique.

La composition selon l'invention peut avoir une brillance moyenne mesurée à 20 °, supérieure ou égale à 35 et/ou une brillance moyenne mesurée à 60 ° supérieure ou égale à 65.

La composition selon l'invention contient avantageusement de 5 à 50 %, en poids, en particulier de 10 à 40 % en poids, de préférence de 15 à 35 % en poids d'au moins une huile brillante, par rapport au poids total de la composition.

## MILIEU PHYSIOLOGIQUEMENT ACCEPTABLE

Par « milieu physiologiquement acceptable », on entend un milieu compatible avec les matières kératiniques, comme les huiles ou les solvants organiques couramment employés dans les compositions cosmétiques.

Selon un mode de mise en oeuvre, le milieu physiologiquement acceptable comprend au moins un corps gras liquide à température ambiante (25 °C en général). Ce corps gras liquide peut être d'origine animale, végétale, minérale ou synthétique.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

Le milieu physiologiquement acceptable de la composition selon l'invention peut également comprendre un

ou plusieurs solvants organiques, physiologiquement acceptables (tolérance, toxicologie et toucher acceptables).

Par solvant de polymérisation, on entend un solvant ou un mélange de solvants. Le solvant de polymérisation du copolymère selon l'invention peut être choisi notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation du copolymère est un mélange acétate de butyle et isopropanol, ou l'isododécane.

Comme solvants utilisables dans la composition de l'invention, on peut citer, outre les solvants de polymérisation cités plus haut, les cétones liquides à température ambiante tels que méthyléthylcétone, méthylisobutylcétone, diisobutylcétone, l'isophorone, la cyclohexanone, l'acétone ; les éthers de propylène glycol liquides à température ambiante tels que le monométhyléther de propylène glycol, l'acétate de monométhyl éther de propylène glycol, le mono n-butyl éther de dipropylène glycol ; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ; les éthers liquides à température ambiante tels que le diéthyléther, le diméthyléther ou le dichlorodiéthyléther ; les alcanes liquides à température ambiante tels que le décane, l'heptane, le dodécane, l'isododécane, le cyclohexane ; les composés cycliques aromatiques liquides à température ambiante tels que le toluène et le xylène ; les aldéhydes liquides à température ambiante tels que le benzaldéhyde, l'acétaldéhyde et leurs mélanges.

Le milieu physiologiquement acceptable peut comprendre, un milieu hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en $C_2$ et des aldéhydes en $C_2$-$C_4$ hydrophiles.

La composition selon l'invention peut également être sensiblement exempte d'un tel milieu hydrophile. La composition selon l'invention peut comprendre moins de 5 % en poids, voire moins de 2 % en poids d'eau par rapport au poids total de la composition et peut en particulier être anhydre.

La composition peut comprendre, outre le copolymère décrit précédemment, un polymère additionnel tel qu'un polymère filmogène.

Selon la présente invention, on entend par « polymère filmogène », un polymère apte à former à lui seul ou en présence d'un agent auxiliaire de filmification, un film continu et adhérent sur un support, notamment sur les matières kératiniques.

Parmi les polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

Le polymère peut être associé à un ou des agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition selon l'invention peut également comprendre au moins une cire.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

La nature et la quantité des corps gras solides sont fonction des propriétés mécaniques et des textures recherchées.

A titre indicatif, la composition peut contenir de 0 à 50 % en poids de cires, par rapport au poids total de la composition et mieux de 1 à 30 % en poids.

Une composition cosmétique conforme à la présente invention peut également comprendre au moins un composé pâteux.

Par composé pâteux on entend notamment un composé gras à changement d'état solide/liquide réversible et comportant à la température de 23 °C une fraction liquide et une fraction solide.

Le composé pâteux peut être choisi parmi :

- la lanoline et ses dérivés
- les composés siliconés polymères ou non
- les composés fluorés polymères ou non
- les polymères vinyliques
- les polyéthers liposolubles résultant de la polyéthérification entre un ou plusieurs diols en $C_2$-$C_{100}$, de préférence en $C_2$-$C_{50}$,
- les esters,
- et leurs mélanges.

La composition selon l'invention peut en outre comprendre un gélifiant lipophile.

Ils peut en particulier s'agir de gélifiants lipophiles, organiques ou minéraux, polymériques ou moléculaires.

Comme gélifiants lipophiles, on peut citer les argiles, éventuellement modifiées comme les hectorites modifiées, la silice traitée hydrophobe, les sels métalliques d'acides gras comme les stéarates d'aluminium, et leurs mélanges.

La composition selon l'invention peut en outre comprendre une matière colorante choisie parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

La composition selon l'invention peut comprendre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être destinée au soin et/ou au maquillage des matières kératiniques, notamment des lèvres et de la peau, en particulier des lèvres.

La composition selon l'invention peut être sous forme d'un gloss liquide.

Les exemples qui suivent illustrent de manière non limitative une composition selon l'invention.

Les quantités sont exprimées en gramme.

## EXEMPLES

### Exemple 1 : préparation d'un copolymère de poly (acrylate d'isobornyle / méthacrylate disobomyle / acrylate isobutyle / acide acrylique).

300 g d'isododécane sont introduits dans un réacteur de 1 litre, puis on augmente la température de façon à passer de la température ambiante (25 °C) à 90 °C en 1 heure.

On ajoute ensuite, à 90 °C et en 1 heure, 105 g de méthacrylate d'isobornyle, 105 g d'acrylate d'isobornyle et 1,8 g de 2.5- Bis(2-éthylhexanoylperoxy)-2.5-diméthylhexane (Trigonox® 141 d'Akzo Nobel).

Le mélange est maintenu lh30 à 90 °C.

On introduit ensuite au mélange précédent, toujours à 90°C et en 30 minutes, 75 g d'acrylate d'isobutyle, 15 g d'acide acrylique et 1,2 g de 2.5-Bis (2-ethylhexanoylperoxy)-2.5-diméthylhexane.

Le mélange est maintenu 3 heures à 90 °C, puis l'ensemble est refroidi.

On obtient une solution à 50 % de matière active en copolymère dans l'isododécane.

On obtient un copolymère comprenant une première séquence ou bloc poly(acrylate d'isobornyle/méthacrylate d'isobornyle) ayant une Tg de 128 °C, une deuxième séquence poly (acrylate d'isobutyle/acide acrylique) ayant une Tg de -9 °C et une séquence intermédiaire qui est un copolymère statistique acrylate d'isobornyle/méthacrylate d'isobornyle/acrylate d'isobutyle/acide acrylique.

La Tg du copolymère est de 74 °C.

Il s'agit de Tg théoriques calculées par la loi de Fox

### Exemple 2 : Formulation de type gloss liquide

Le mode opératoire pour 200g de la formulation suivante est le suivant :

• Les pigments sont broyés 3 fois à la broyeuse tricylindre dans l'octyldodécanol porté au préalable à 60 °C. Le broyat est laissé à refroidir à température ambiante (25 °C) dans un poêlon double paroi ou un bécher.
• Le copolymère , le squalane, le polybutylène, les nacres et le parfum sont ajoutés au broyat. Le tout est agité à la turbine (type : Rayneri) pour homogénéiser.
• Quand le mélange est homogène, la polyphenyl trimethylsiloxy dimethylsiloxane est ajoutée sous agitation à 800 tours/minute au Rayneri pendant 30 minutes environ.
• Enfin, la sililce pyrogénée est ajoutée en pluie et l'agitation à la turbine est maintenue à 1000 tours/minute pendant 20 minutes.

| NOM | Concentration (% massique) |
|---|---|
| Perhydrosqualene végétal raffinée (Nom INCI = squalane) | 10,86 |
| Octyl-2 dodécanol | 15,39 |
| Oxyde de titane rutile traité aluminé/silice/trimethyolpropane | 2,74 |
| RED 7 | 0,54 |
| Lake Blue 1 | 0,16 |
| Lake Yellow 6 | 2,58 |
| Oxyde de fer noir | 0,25 |
| Mica - dioxyde de titane - oxyde de fer brun | 2 |
| Polyphenyltrimethylsiloxy dimethylsiloxane [1] | 20,03 |
| Silice pyrogénée hydrophobe, traitée en surface par diméthylsilane [3] | 4,5 |

(suite)

| NOM | Concentration (% massique) |
|---|---|
| Poly(méthacrylate d'isobornyle-co-acrylate d'isobornyle-co-acrylate d'isobutyle-co-acide acrylique) à 50 % en matière active dans l'isododécane | 30 |
| Polybutylène [2] | 10,65 |
| Parfum | 0,3 |
| Total | 100 |
| [1] : Belsil PDM 1000 de WACKER (viscosité 1000 cPs PM : 9000)<br>[2] : INDOPOL H 100 (PM : 920)<br>[3] : AEROSIL R 972 de DEGUSSA | |

Cette composition de gloss, appliquée sur les lèvres en un seul geste, présente des propriétés de confort et de brillance satisfaisantes.

La tenue de la composition est également améliorée ; la composition ne migre pas dans les rides et ridules du contour des lèvres.

## Revendications

**1.** Composition cosmétique comprenant, dans un milieu physiologiquement acceptable, au moins :

- un copolymère séquencé, comprenant au moins une première séquence et au moins une deuxième séquence,

  - la première séquence étant obtenue à partir d'au moins un monomère acrylate de formule CH2 = CH-COOR2 dans laquelle R2 représente un groupe cycloalkyle C4 à C12 et d'au moins un monomère méthacrylate de formule CH2 = C(CH3)-COOR'2 dans laquelle R'2 représente un groupe cycloalkyle C4 à C12,
  - la deuxième séquence étant obtenue à partir d'un monomère acide acrylique et d'au moins un autre monomère de transition vitreuse inférieure ou égale à 20°C choisi parmi,

- les acrylates de formule CH2 = CHCOOR3, R3 représentant un groupe alkyle non substitué en C1 à C12, linéaire ou ramifié, à l'exception du groupe tertiobutyle, dans lequel se trouve(nt) éventuellement intercalé(s) un ou plusieurs hétéroatomes choisis parmi O, N, S, ,
- les méthacrylates de formule CH2 = C(CH3)-COOR4, R4 représentant un groupe alkyle non substitué en C6 à C12 linéaire ou ramifié, dans lequel se trouve(nt) éventuellement intercalé(s) un ou plusieurs hétéroatomes choisis parmi O, N et S;
- les esters de vinyle de formule R5-CO-O-CH = CH2 où R5 représente un groupe alkyle en C4 à C12 linéaire ou ramifié ;
- les éthers d'alcool vinylique et d'alcool en C4 à C12,
- les N-alkyl en C4 à C12 acrylamides, tels que le N-octylacrylamide,
- et leurs mélanges,

  - une huile non volatile ayant une chaine hydrocarbonée comprenant au moins 16 atomes de carbone et ayant une masse molaire inférieure à 650 g/mole, et
  - une huile brillante ayant une masse molaire allant de 650 à 10000 g/mole choisie parmi les polybutylènes, les polyisobutylènes hydrogénés, les polydécènes et les polydécènes hydrogénés, les copolymères de la vinylpyrrolidone, les esters d'acide gras linéaires ayant un nombre total de carbone allant de 35 à 70, les esters hydroxylés, le tridécyl trimellitate, les esters d'alcool gras ou d'acide gras ramifiés en $C_4$-$C_{28}$, les huiles siliconées phénylées, l'huile de sésame et leurs mélanges .

**2.** Composition selon la revendication précédente, dans laquelle le copolymère comprend plus de 2 % en poids de monomères acide acrylique, et notamment de 2 à 15 % en poids, par exemple de 3 à 15 % en poids, en particulier de 4 à 15 % en poids, voire de 5 à 10 % en poids de monomères acide acrylique, par rapport au poids total dudit copolymère.

3. Composition selon l'une quelconque des revendications précédentes, dans laquelle R2 et R'2 représentent indépendamment ou simultanément un groupe isobornyle.

4. Composition selon l'une quelconque des revendications précédentes, dans laquelle la proportion de la première séquence va de 20 à 90 % en poids par rapport au poids total du copolymère, mieux de 30 à 80 % en poids et encore mieux de 60 à 80 % en poids.

5. Composition selon l'une quelconque des revendications précédentes, dans laquelle le monomère de transition vitreuse inférieure ou égale à 20 °C est choisi parmi des acrylates de formule $CH2 = CHCOOR3$, avec R3 représentant un groupe alkyle non substitué en C1 à C12, linéaire ou ramifié, à l'exception du groupe tertiobutyle, dans lequel se trouve(nt) éventuellement intercalé(s) un ou plusieurs hétéroatomes choisis parmi O, N, S.

6. Composition selon la revendication 5, dans laquelle R3 représente l'isobutyle.

7. Composition selon l'une quelconque des revendications précédentes, dans laquelle le copolymère comprend au moins des monomères acrylate d'isobornyle et méthacrylate d'isobornyle dans la première séquence et des monomères acrylate d'isobutyle et acide acrylique dans la deuxième séquence.

8. Composition selon l'une quelconque des revendications précédentes, dans laquelle ledit copolymère comprend de 50 à 80 % en poids de méthacrylate/acrylate d'isobornyle, de 10 à 30 % en poids d'acrylate d'isobutyle et de 2 à 10 % en poids d'acide acrylique.

9. Composition selon l'une quelconque des revendications précédentes, comprenant moins de 40 % en poids de matière active de copolymère, et avantageusement de 5 à 40 % en poids, notamment de 5 à 30 % en poids, voire de 10 à 20 % en poids, par rapport au poids total de la composition.

10. Composition selon l'une quelconque des revendications précédentes, dans laquelle l'huile non volatile comprend une huile ayant une viscosité comprise entre 10 et 300 cPs, de préférence entre 15 et 200 cPs.

11. Composition selon l'une quelconque des revendications précédentes, dans laquelle l'huile non volatile comprend une huile hydrocarbonée, linéaire ou ramifiée ayant une masse molaire comprise entre 100 et 650 g/mol et plus particulièrement entre 200 et 650 g/mol.

12. Composition selon l'une quelconque des revendications précédentes, dans laquelle l'huile non volatile est choisie parmi les polyalcènes.

13. Composition selon la revendication précédente, dans laquelle les polyalcènes sont en particulier les polybutylènes, les polydécènes, les alcanes et en particulier le squalane, les isoparaffines hydrogénées, l'isoeicosane et leurs mélanges.

14. Composition selon l'une quelconque des revendications précédentes, dans laquelle l'huile non volatile est le squalane.

15. Composition selon l'une quelconque des revendications précédentes, comprenant de 2 à 50 % en poids d'huile non volatile, notamment de 5 à 20 % en poids par rapport au poids total de la composition.

16. Composition selon l'une quelconque des revendications précédentes, dans laquelle l'huile brillante a une masse molaire allant de 750 à 7 500 g/mole.

17. Composition selon l'une quelconque des revendications précédentes, dans laquelle l'huile brillante est du polybutylène.

18. Composition selon l'une quelconque des revendications précédentes, comprenant de 5 à 50 % en poids, en particulier de 10 à 40 % en poids, de préférence de 15 à 35 % en poids d'au moins une huile brillante, par rapport au poids total de la composition.

19. Composition selon l'une quelconque des revendications précédentes, ayant une brillance moyenne mesurée à 20 °, supérieure ou égale à 35 et/ou une brillance moyenne mesurée à 60 ° supérieure ou égale à 65.

**20.** Composition selon l'une quelconque des revendications précédentes comprenant en outre une matière colorante.

**21.** Composition selon l'une quelconque des revendications précédentes, celle-ci étant anhydre.

**22.** Composition selon l'une quelconque des revendications précédentes, destinée au soin et/ou au maquillage des lèvres.

**23.** Composition selon l'une quelconque des revendications précédentes, sous forme d'un gloss liquide.

**24.** Procédé cosmétique de maquillage des lèvres comprenant l'application sur les lèvres d'une composition telle que définie selon l'une quelconque des revendications précédentes.

**25.** Utilisation d'un copolymère séquencé, comprenant au moins une première séquence et au moins une deuxième séquence,
la première séquence étant obtenue à partir d'au moins un monomère acrylate de formule $CH_2 = CH\text{-}COOR_2$ dans laquelle $R_2$ représente un groupe cycloalkyle $C_4$ à $C_{12}$ et d'au moins un monomère méthacrylate de formule $CH_2 = C(CH_3)\text{-}COOR'_2$ dans laquelle $R'_2$ représente un groupe cycloalkyle $C_4$ à $C_{12}$,
la deuxième séquence étant obtenue à partir d'un monomère acide acrylique et d'au moins un autre monomère de transition vitreuse inférieure ou égale à 20°C choisi parmi,

- les acrylates de formule CH2 = CHCOOR3, R3 représentant un groupe alkyle non substitué en C1 à C12, linéaire ou ramifié, à l'exception du groupe tertiobutyle, dans lequel se trouve(nt) éventuellement intercalé(s) un ou plusieurs hétéroatomes choisis parmi O, N, S, ,
- les méthacrylates de formule CH2 = C(CH3)-COOR4, R4 représentant un groupe alkyle non substitué en C6 à C12 linéaire ou ramifié, dans lequel se trouve(nt) éventuellement intercalé(s) un ou plusieurs hétéroatomes choisis parmi O, N et S;
- les esters de vinyle de formule R5-CO-O-CH = CH2 où R5 représente un groupe alkyle en C4 à C12 linéaire ou ramifié ;
- les éthers d'alcool vinylique et d'alcool en C4 à C12,
- les N-alkyl en C4 à C12 acrylamides, tels que le N-octylacrylamide,
- et leurs mélanges,

dans une composition destinée à procurer un dépôt sur les lèvres, doté d'une bonne tenue et d'une brillance satisfaisante.

**26.** Utilisation selon la revendication précédente dans laquelle la composition est telle que définie dans l'une quelconque des revendications 1 à 23.

**27.** Utilisation selon la revendication 25 ou 26, dans laquelle le copolymère est tel que défini en revendications 1 à 9.

## Patentansprüche

**1.** Kosmetische Zusammensetzung, umfassend, in einem physiologisch verträglichen Medium, mindestens folgendes: ein Blockcopolymer, umfassend mindestens einen ersten Block und mindestens einen zweiten Block,

- wobei der erste Block ausgehend von mindestens einem Acrylatmonomer der Formel CH2 = CH-COOR2, wobei R2 eine C4-C12-Cycloalkylgruppe darstellt, und mindestens einem Methacrylatmonomer der Formel CH2 = C(CH3)-COOR'2, wobei R' 2 eine C4 bis C12 Cycloalkylgruppe darstellt, erhalten wird,
- wobei der zweite Block aus einem Acrylsäuremonomer und mindestens einem anderen Monomer mit einer Glasübergangstemperatur von weniger als oder gleich 20°C erhalten wird, ausgewählt aus,

  - Acrylaten der Formel CH2 = CHCOOR3, wobei R3 eine lineare oder verzweigte unsubstituierte C1-C12-Alkylgruppe, mit Ausnahme der Tertiärbutylgruppe, darstellt, in der gegebenenfalls ein oder mehrere Heteroatome ausgewählt aus O, N, S eingebaut ist (sind),
  - Methacrylaten der Formel CH2 = C(CH3)-COOR4, wobei R4 eine unsubstituierte, lineare oder verzweigte C6-C12-Alkylgruppe darstellt, in der gegebenenfalls ein oder mehrere Heteroatome ausgewählt aus O, N, S eingebaut ist (sind);

- Vinylestern der Formel R5-CO-O-CH = CH2, wobei R5 eine lineare oder verzweigte C4-C12 Alkylgruppe darstellt;
- Vinylalkohol- und C4-C12-Alkoholethern,
- C4-C12-N-Alkylacrylamiden, wie z.B. N-Octylacrylamid,
- und ihren Gemischen,

- ein nichtflüchtiges Öl mit einer Kohlenwasserstoffkette, die mindestens 16 Kohlenstoffatome umfasst und ein Molekulargewicht von weniger als 650 g/mol aufweist, und
- ein Glanzöl mit einem Molekulargewicht im Bereich von 650 bis 10000 g/mol, ausgewählt aus Polybutylenen, hydrierten Polyisobutylenen, hydrierten Polydecenen und Polydecenen, Vinylpyrrolidon-Copolymeren, linearen Fettsäureestern mit einer Gesamtkohlenstoffzahl von 35 bis 70, Hydroxylestern, Tridecyltrimellitat, Fettalkohol- oder verzweigten C4-C28-Fettsäureestern, phenylhaltigen Silikonölen, Sesamöl und ihren Gemischen.

**2.** Zusammensetzung nach vorangehendem Anspruch, wobei das Copolymer mehr als 2 Gew.-% Acrylsäuremonomere und insbesondere 2 bis 15 Gew.-%, beispielsweise 3 bis 15 Gew.-%, insbesondere 4 bis 15 Gew.-%, oder sogar 5 bis 10 Gew.-% Acrylsäuremonomere, bezogen auf das Gesamtgewicht des Copolymers, umfasst.

**3.** Zusammensetzung nach einem der vorangehenden Ansprüche, wobei R2 und R'2 unabhängig oder gleichzeitig eine Isobornylgruppe darstellen.

**4.** Zusammensetzung nach einem der vorangehenden Ansprüche, wobei der Anteil des ersten Blocks 20 bis 90 Gew.-%, bezogen auf das Gesamtgewicht des Copolymers, besser 30 bis 80 Gew.-% und noch besser 60 bis 80 Gew.-% beträgt.

**5.** Zusammensetzung nach einem der vorangehenden Ansprüche, wobei das Monomer mit einer Glasübergangstemperatur von kleiner oder gleich 20°C ausgewählt ist aus Acrylaten der Formel CH2 = CHCOOR3, wobei R3 eine lineare oder verzweigte unsubstituierte Alkylgruppe mit 1 bis 12 Kohlenstoffatomen, mit Ausnahme der Tertiärbutylgruppe, darstellt, in die gegebenenfalls ein oder mehrere Heteroatome ausgewählt aus O, N, S eingebaut sind.

**6.** Zusammensetzung nach Anspruch 5, wobei R3 Isobutyl darstellt.

**7.** Zusammensetzung nach einem der vorangehenden Ansprüche, wobei das Copolymer mindestens Isobornylacrylat- und Isobornylmethacrylatmonomere im ersten Block und Isobutylacrylat- und Acrylsäuremonomere im zweiten Block umfasst.

**8.** Zusammensetzung nach einem der vorangehenden Ansprüche, wobei das Copolymer 50 bis 80 Gew.-% Isobornylmethacrylat/Acrylat, 10 bis 30 Gew.-% Isobutylacrylat und 2 bis 10 Gew.-% Acrylsäure umfasst.

**9.** Zusammensetzung nach einem der vorangehenden Ansprüche, umfassend weniger als 40 Gew.-% aktives Copolymer-Material und vorteilhafterweise 5 bis 40 Gew.-%, einschließlich 5 bis 30 Gew.-%, oder sogar 10 bis 20 Gew.-%, bezogen auf das Gesamtgewicht der Zusammensetzung.

**10.** Zusammensetzung nach einem der vorangehenden Ansprüche, wobei das nichtflüchtige Öl ein Öl mit einer Viskosität von 10 bis 300 cPs, vorzugsweise 15 bis 200 cPs, umfasst.

**11.** Zusammensetzung nach einem der vorangehenden Ansprüche, wobei das nichtflüchtige Öl ein lineares oder verzweigtes Kohlenwasserstofföl mit einem Molekulargewicht von 100 bis 650 g/mol und insbesondere von 200 bis 650 g/mol umfasst.

**12.** Zusammensetzung nach einem der vorangehenden Ansprüche, wobei das nichtflüchtige Öl aus Polyalkenen ausgewählt ist.

**13.** Zusammensetzung nach dem vorangehenden Anspruch, wobei die Polyalkene insbesondere Polybutylene, Polydecene, Alkane, und insbesondere Squalan, hydrierte Isoparafine, Isoeicosan und Mischungen davon sind.

**14.** Zusammensetzung nach einem der vorangehenden Ansprüche, wobei das nichtflüchtige Öl Squalan ist.

**15.** Zusammensetzung nach einem der vorangehenden Ansprüche, umfassend 2 bis 50 Gew.-% nichtflüchtiges Öl,

insbesondere 5 bis 20 Gew.-%, bezogen auf das Gesamtgewicht der Zusammensetzung.

**16.** Zusammensetzung nach einem der obigen Ansprüche, wobei das Glanzöl ein Molekulargewicht im Bereich von 750 bis 7.500 g/mol aufweist.

**17.** Zusammensetzung nach einem der vorangehenden Ansprüche, wobei das Glanzöl Polybutylen ist.

**18.** Zusammensetzung nach einem der vorangehenden Ansprüche, umfassend 5 bis 50 Gew.-%, insbesondere 10 bis 40 Gew.-%, vorzugsweise 15 bis 35 Gew.-% von mindestens einem Glanzöl, bezogen auf das Gesamtgewicht der Zusammensetzung.

**19.** Zusammensetzung nach einem der vorangehenden Ansprüche, mit einer durchschnittlichen Glanzzahl, gemessen bei 20°, von größer oder gleich 35, und/oder einer durchschnittlichen Glanzzahl, gemessen bei 60°, von größer oder gleich 65.

**20.** Zusammensetzung nach einem der vorangehenden Ansprüche, die ferner ein Farbmittel umfasst.

**21.** Zusammensetzung nach einem der vorangehenden Ansprüche, die wasserfrei ist.

**22.** Zusammensetzung nach einem der oben genannten Ansprüche, die zur Pflege und/oder zum Schminken der Lippen bestimmt ist.

**23.** Zusammensetzung nach einem der vorangehenden Ansprüche in Form eines flüssigen Glosses.

**24.** Kosmetisches Lippen-Schminkverfahren, umfassend das Auftragen einer Zusammensetzung, nach einem der vorangehenden Ansprüche auf die Lippen.

**25.** Verwendung eines Blockcopolymers, umfassend mindestens einen ersten Block und mindestens einen zweiten Block, wobei der erste Block ausgehend von mindestens einem Acrylatmonomer der Formel CH2 = CH-COOR2, wobei R2 eine C4 bis C12 Cycloalkylgruppe darstellt, und mindestens einem Methacrylatmonomer der Formel CH2 = C(CH3)-COOR'2 erhalten wird, wobei R' 2 eine C4-C12-Cycloalkylgruppe darstellt, wobei der zweite Block aus einem Acrylsäuremonomer und mindestens einem anderen Monomer mit einer Glasübergangstemperatur von kleiner oder gleich 20°C erhalten wird, das ausgewählt ist aus,

- Acrylaten der Formel CH2 = CHCOOR3, wobei R3 eine lineare oder verzweigte, unsubstituierte C1-C12-Alkylgruppe, mit Ausnahme der Tertiärbutylgruppe, darstellt, in die gegebenenfalls ein oder mehrere Heteroatome ausgewählt aus O, N, S eingebaut sind,
- Methacrylaten der Formel CH2 = C(CH3)-COOR4, wobei R4 eine unsubstituierte lineare oder verzweigte C6-C12-Alkylgruppe darstellt, in die gegebenenfalls ein oder mehrere Heteroatome ausgewählt aus O, N und S eingebaut sind;
- Vinylestern der Formel R5-CO-O-CH = CH2, wobei R5 eine lineare oder verzweigte C4-C12-Alkylgruppe darstellt;
- Vinylalkohol- und C4-C12-Alkoholethern,
- C4-C12-N-Alkylacrylamiden, wie N-Octylacrylamid,
- und deren Mischungen,

in einer Zusammensetzung, die dazu bestimmt ist, eine Ablagerung auf den Lippen mit gutem Halt und zufriedenstellendem Glanz zu schaffen.

**26.** Verwendung nach dem vorangehenden Anspruch, wobei die Zusammensetzung wie in einem der Ansprüche 1 bis 23 definiert ist.

**27.** Verwendung nach Anspruch 25 oder 26, wobei das Copolymer wie in den Ansprüchen 1 bis 9 definiert ist.

**Claims**

**1.** Cosmetic composition comprising, in a physiologically-acceptable medium, at least:

- a block copolymer comprising at least a first block and at least a second block,
- wherein the first block is obtained from at least one acrylate monomer of formula CH2=CH-COOR2 in which R2 represents a C4 to C12 cycloalkyl group and from at least one methacrylate monomer of formula CH2=C(CH3)-COOR'2 in which R'2 represents a C4 to C12 cycloalkyl group,
- wherein the second block is obtained from an acrylic acid monomer and from at least one other monomer with a glass transition of less than or equal to 20°C, and is chosen from:

  - acrylates of formula CH2=CHCOOR3, with R3 representing an unsubstituted, linear or branched C1 to C12 alkyl group, with the exception of the tert-butyl group, in which one or more heteroatoms chosen from O, N and S is (are) optionally intercalated,
  - methacrylates of formula CH2=C(CH3)-COOR4, R4 representing an unsubstituted, linear or branched C6 to C12 alkyl group in which one or more heteroatoms chosen from O, N and S are optionally intercalated,
  - vinyl esters of formula R5-CO-O-CH=CH2 where R5 represents a linear or branched C4 to C12 alkyl group,
  - ethers of vinyl alcohol and of a C4 to C12 alcohol,
  - N-(C4 to C12)alkylacrylamides such as N-octylcrylamide,
  - and mixtures thereof.
  - one non-volatile oil that has a hydrocarbon-based chain comprising at least 16 carbon atoms and having a molar mass of less than 650 g/mol, and
  - a glossy oil having a molecular weight ranging from 650 to 10,000 g / mol chosen from polybutylenes, hydrogenated polyisobutylenes, hydrogenated polydecenes and polydecenes, copolymers of vinylpyrrolidone, and esters of linear fatty acids having a total carbon number ranging from 35 to 70; hydroxylated esters, tridecyl trimellitate, branched C4-C28 fatty alcohol or fatty acid esters, phenyl silicone oils, sesame oil and mixtures thereof

2. Composition according to the preceding claim, wherein the copolymer comprises more than 2% by weight of acrylic acid monomers, and in particular from 2 to 15% by weight, for example from 3 to 15% by weight, in particular from 4 to 15% by weight, or even 5 to 10% by weight of acrylic acid monomers, relative to the total weight of the copolymer

3. Composition according to any one of the preceding claims, wherein R2 and R'2 independently or simultaneously represent an isobornyl group.

4. Composition according to any one of the preceding claims, wherein the proportion of the first block is from 20 to 90% by weight relative to the total weight of the copolymer, more preferably 30 to 80% by weight, and even more preferably 60 to 80% by weight.

5. Composition according to any of the preceding claims, wherein the monomer with a glass transition of less than or equal to 20°C is chosen from acrylates of formula CH2=CHCOOR3, with R3 representing an unsubstituted linear or branched C1 to C12 alkyl group, with the exception of the tert-butyl group, wherein one or more heteroatoms chosen from O, N and S are optionally intercalated.

6. Composition according to the claim 5, wherein R3 represents isobutyl.

7. Composition according to any one of the preceding claims, wherein the copolymer comprises at least isobornyl acrylate and isobornyl methacrylate monomers in the first block and isobutyl acrylate and acrylic acid monomers in the second block.

8. Composition according to any one of the preceding claims, wherein the copolymer comprises from 50% to 80% by weight of isobornyl methacrylate/acrylate, from 10% to 30% by weight of isobutyl acrylate, and from 2% to 10% by weight of acrylic acid.

9. Composition according to any one of the preceding claims, comprising less than 40% by weight of copolymer active material, and advantageously from 5 to 40% by weight, in particular from 5 to 30% by weight, and even from 10 to 20% by weight, relative to the total weight of the composition

10. Composition according to any one of the preceding claims, wherein the non-volatile oil comprises an oil having a viscosity between 10 and 300 cPs, preferably between15 and 200 cPs.

11. Composition according to any one of the preceding claims, wherein the non-volatile oil comprises a linear or branched,

hydrocarbon-based oil having a molar mass of between 100 and 650 g/mol, and more particularly between 200 and 650 g/mol.

**12.** Composition according to any one of the preceding claims, wherein the non-volatile oil is chosen from polyalkenes.

**13.** Composition according to any one of the preceding claims, wherein the polyalkenes are chosen from polybutylenes, polydecenes, alkanes, and, in particular, squalane, hydrogenated isoparaffins, isoeicosane, and mixtures thereof.

**14.** Composition according to any one of the preceding claims, wherein the non-volatile oil is squalane.

**15.** Composition according to any one of the preceding claims, comprising from 2% to 50% by weight of non-volatile oil, in particular 5 to 20% by weight relative to the total weight of the composition.

**16.** Composition according to any of the preceding claims, wherein the glossy oil has a molar mass ranging from 750 to 7 500 g/mol.

**17.** Composition according to any one of the preceding claims, wherein the glossy oil is polybutylene.

**18.** Composition according to any one of the preceding claims, comprising from 5% to 50% by weight, in particular from 10 to 40% by weight, preferably from 15 to 35% by weight of at least one glossy oil, relative to the total weight of the composition.

**19.** Composition according to any one of the preceding claims, having an average gloss, measured at 20°, of greater than or equal to 35 and/or an average gloss, measured at 60°, of greater than or equal to 65.

**20.** Composition according to any one of the preceding claims, also comprising a dyestuff.

**21.** Composition according to any one of the preceding claims, wherein the composition is anhydrous.

**22.** Composition according to any one of the preceding claims for caring for and/or making up the lips.

**23.** Composition according to any one of the preceding claims, in the form of a liquid gloss.

**24.** Cosmetic process for making up the lips, comprising the application to the lips of a composition as defined according to any one of the preceding claims

**25.** Use of a block copolymer comprising at least a first block and at least a second block,
wherein the first block is obtained from at least one acrylate monomer of formula $CH_2$=CH-$COOR_2$ in which $R_2$ represents a C4 to C12 cycloalkyl group and from at least one methacrylate monomer of formula $CH_2$=C($CH_3$)-CO-$OR'_2$ in which $R'_2$ represents a C4 to C12 cycloalkyl group,
wherein the second block is obtained from an acrylic acid monomer and from at least one other monomer with a glass transition of less than or equal to 20°C, and is chosen from:

- acrylates of formula CH2=CHCOOR3, with R3 representing an unsubstituted, linear or branched C1 to C12 alkyl group, with the exception of the tert-butyl group, in which one or more heteroatoms chosen from O, N and S is (are) optionally intercalated,
- methacrylates of formula CH2=C(CH3)-COOR4, R4 representing an unsubstituted, linear or branched C6 to C12 alkyl group in which one or more heteroatoms chosen from O, N and S are optionally intercalated,
- vinyl esters of formula R5-CO-O-CH=CH2 where R5 represents a linear or branched C4 to C12 alkyl group,
- ethers of vinyl alcohol and of a C4 to C12 alcohol,
- N-(C4 to C12)alkylacrylamides such as N-octylcrylamide,
- and mixtures thereof.

in a composition intended to obtain a deposit on the lips featuring good holding and a satisfactory gloss.

**26.** Use according to the preceding claim, wherein the composition is as defined in any one of the claims 1 to 23.

**27.** Use according to claim 25 or 26, wherein the copolymer is as defined in the claims 1 to 9.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2880268 A2
- EP 1604634 A1
- WO 02067877 A
- EP 1518534 A
- EP 0955039 A


### Littérature non-brevet citée dans la description


- Polymer Handbook. John Wiley, 1989